(19)

Europäisches
Patentamt
European
Patent Office
Office européen
des brevets

(11) **EP 4 295 797 B1**

(12) **EUROPEAN PATENT SPECIFICATION**

(45) Date of publication and mention
of the grant of the patent:
**03.09.2025 Bulletin 2025/36**

(21) Application number: **23180702.5**

(22) Date of filing: **21.06.2023**

(51) International Patent Classification (IPC):
***A61B 18/14*** (2006.01)    *A61B 18/12* (2006.01)
***A61B 18/00*** (2006.01)

(52) Cooperative Patent Classification (CPC):
**A61B 18/1492;** A61B 18/1233; A61B 2018/00351;
A61B 2018/00577; A61B 2018/00613;
A61B 2018/00791; A61B 2018/00875;
A61B 2018/1467

(54) **PROJECTION OF PULSED ELECTRIC FIELD (PEF) INDEX**

PROJEKTION DES INDEX EINES GEPULSTEN ELEKTRISCHEN FELDES (PEF)

PROJECTION D'INDICE DE CHAMP ÉLECTRIQUE PULSÉ (PEF)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **22.06.2022 US 202263354334 P
12.06.2023 US 202318332953**

(43) Date of publication of application:
**27.12.2023 Bulletin 2023/52**

(73) Proprietor: **Medtronic Inc.
Minneapolis, MN 55432 (US)**

(72) Inventors:
• **Howard, Brian T.
Minneapolis, MN 55432 (US)**
• **Schmidt, Megan M.
Minneapolis, MN 55432 (US)**
• **Mattison, Lars M.
Minneapolis, MN 55432 (US)**

(74) Representative: **Zimmermann & Partner
Patentanwälte mbB
Postfach 330 920
80069 München (DE)**

(56) References cited:
**US-A1- 2020 060 757    US-A1- 2020 261 150
US-A1- 2021 186 604**

**Description**

FIELD

The present disclosure relates to methods, systems, and devices for enhancing the efficiency and efficacy of ablation energy delivery and improving patient safety.

BACKGROUND

Tissue ablation is used in numerous medical procedures to treat a patient. In some examples, ablation procedures involve modification of target tissue, e.g., to stop electrical propagation through the tissue in patients with an arrhythmia. Such ablation procedures are often performed by passing energy, such as electrical energy, through one or more electrodes of an inserted catheter. The energy causes modifications to the target tissue.
US 2020/060757 A1 discloses estimators for ablation effectiveness.

SUMMARY OF THE INVENTION

The invention is defined by the independent claims. In the following all methods relating to treatment of the human body are disclosed but not claimed and are shown for illustrative purposes only.

SUMMARY OF THE DISCLOSURE.

Medical procedures, such as cardiac ablation using one or more energy modalities are frequently used to treat conditions such as atrial fibrillation and ventricular tachycardia. Some treatments may use radiofrequency ablation (RF) which heats target tissue in the heart to cause cell death and thus change conduction pathways in the heart to treat the disease state in a patient. Excessive application of RF energy can result in collateral damage. To help avoid collateral damage, the desired end to a treatment is assessed by using measurements such as temperature rise, contact force, total energy, and/or impedance measures for example to inform what thermal energy has been conveyed to and retained by the target tissue. Thermal transfer can take several seconds to minutes during which the heart and catheters continue to move. In contrast, pulsed electric field (PEF) ablation use an electric field to disrupt cellular membranes. The pulsed electric field can be generated with electrical pulses delivered in short bursts. The compromised cell membrane results in the desired cell death and is associated with other measurable phenomena, such as impedance changes resulting from the liberated exchange of ions through the permeabilized cell membranes. Different cell types may also be affected differently by PEF energy such that collateral structures typically affected by temperature rise induced by RF ablation may remain unaffected by PEF energy. The temperature rise should not be ignored for PEF ablation entirely however. The electric field in PEF ablation is established between conductive elements, such as electrodes, and currents flowing through the target tissue, which act as a resistive medium, result in energy dissipation and some temperature rise in the tissue and fluid. This temperature rise is generally smaller than the RF-induced temperature rises but still presents a risk to collateral structures, especially with several successive applications.

The techniques of this disclosure generally relate to assessment, mapping, and projection of pulsed electric field (PEF) energy delivery, and in some examples, to a correlation of a PEF field distribution based on the delivery of PEF energy to the target tissue and at least one metric of a therapeutic effect of the delivered PEF energy.

In one aspect, the present disclosure provides for a medical system. The medical system includes a generator configured to generate pulsed electric field (PEF) energy. A medical device is in electrical communication with the generator and has a plurality of electrodes configured to deliver the generated PEF energy to a target tissue to create electroporated regions in the target tissue. A delivery element tracking system is in communication with the generator and the medical device. The tracking system has processing circuitry configured to: measure a position of at least one of the plurality of electrodes proximate in time to a delivery of PEF energy to the target tissue with respect to the target tissue and correlate a PEF field distribution based on the delivery of PEF energy to the target tissue to determine or modify at least one metric of a therapeutic effect from the PEF delivery at positions other than the measured location of the plurality of electrodes.

In another aspect, the tracking system is further configured to correlate the determined at least one metric of a therapeutic effect into one or more zones.

In another aspect, the one or more zones are visually coded to represent a predetermined at least one metric of a therapeutic effect.

In another aspect, the tracking system is further configured to display the visually coded one or more zones on a display overlaying the target tissue.

In another aspect, the one or more zones are representative of thermal damage.

In another aspect, the one or more zones represent a region of tissue that has recovered from reversible effects.

In another aspect, the one or more zones are represented predictively based on set of anticipated PEF parameters and position data of electrodes delivering the PEF energy.

In another aspect, a first one of the visually coded one or more zones is indicated of a first region of the target tissue irreversibly electroporated and a second one of the visually coded one or more zones is indicated of second region of the target tissue reversibly electroporated.

In another aspect, the determined at least one metric of a therapeutic effect is determined based at least in part on at least one from the group consisting of a temperature of the plurality of electrodes during delivery of PEF energy, an impedance of the plurality of electrodes during delivery of PEF energy, and a proximity of the plurality of electrodes to the target tissue.

In another aspect, the at least one metric for therapeutic effect is a measure of the at least one metric of a therapeutic effect compared against a predetermined threshold.

In another aspect, the tracking system is further configured to display the at least one metric of a therapeutic effect on a display.

In another aspect, the displayed at least one metric for therapeutic effect is overlayed on a display of the target tissue.

In another aspect, the delivered PEF energy is sufficient to irreversibly electroporate at least a portion of the target tissue.

In one aspect, a method of determining a region of therapeutic effect in a pulsed electric field (PEF) energy delivery system includes measuring a position of at least one of a plurality of electrodes of a medical device prior to or after delivery of PEF energy to a target tissue with respect to the target tissue. PEF energy is then delivered to the target tissue sufficient to electroporate at least a portion of the target tissue. At least one metric of a therapeutic effect is determined or modified based at least in part on the measured position.

In another aspect, the method further includes correlating the determined at least one metric of a therapeutic effect into one or more zones.

In another aspect, the one or more zones are visually coded to represent the at least one metric of a therapeutic effect, and wherein the method further includes displaying the visually coded one or more zones on a display overlaying the target tissue.

In another aspect, the at least one metric of a therapeutic effect is based at least in part on a fidelity of the measured position of the plurality of electrodes.

In another aspect, a first one of the visually coded one or more zones is indicative of a first region of the target tissue irreversibly electroporated and a second one of the visually coded one or more zones is indicative of second region of the target tissue reversibly electroporated.

In another aspect, at least one metric of a therapeutic effect is determined based at least in part on at least one from the group consisting of a temperature of the plurality of electrodes during delivery of PEF energy, an impedance of the plurality of electrodes during delivery of PEF energy, and a proximity of the plurality of electrodes to the target tissue.

In another aspect, the at least one metric of a therapeutic effect is a measure of the at least one metric of a therapeutic effect compared against a predetermined threshold.

In another aspect, the method further includes displaying the at least one metric of a therapeutic effect on a display.

In another aspect, the displayed at least one metric of a therapeutic effect is overlayed on a display of the target tissue.

In another aspect, the measured position includes multiple independent measurements proximate in time to deliveries of PEF energy.

In another aspect, the measured position is predicted from cyclic, algorithmic, or patterned behavior in the measurements at the time of delivery of PEF energy.

In one aspect, a medical system includes a generator configured to generate pulsed electric field (PEF) energy. A first medical device is in electrical communication with the generator and has a plurality of electrodes configured to deliver the generated PEF energy to a target tissue to create a lesion in the target tissue. A second medical device has a plurality of electrodes and is configured to map the target tissue for at least one from the group consisting of geometric representation, spatial navigation, electrical signals, and temporal behavior of electrical signals. A tracking system is in communication with the generator, the first medical device, and the second medical device. The tracking system has processing circuitry configured to: measure a position of at least one of the plurality of electrodes proximate in time to a delivery of PEF energy to the target tissue with respect to the target tissue with the second medical device; and determine or modify at least one metric of a therapeutic effect based at least in part on the measured position.

In one aspect, a representation of the effect of a specified PEF delivery may be represented concurrent with the modeled space included to the already computed effects from previously completed PEF deliveries to be used as a predictive guide.

In another aspect, zones may be defined in spatial modeling application as points, volumes, or surfaces as practical representations and or addressable elements.

One example provides a medical treatment apparatus. The apparatus includes a catheter having a plurality of electrodes configured to deliver PEF energy to a target tissue and further configured for therapy assessment measurements in the target tissue, the therapy assessment measurements including a first measurement taken prior to a respective time instance of PEF-energy delivery and a second measurement taken after the respective time instance. The apparatus also includes a tracking system configured to track a position of a selected electrode of the plurality of electrodes or of a selected tracking element to determine a first displacement of the plurality of electrodes with respect to the target tissue between the first measurement and the respective time instance and a second displacement of the plurality of electrodes with respect to the target tissue between the respective time instance and the second measurement. The apparatus further includes a processing circuit configured to estimate a therapeutic effect of the PEF-energy delivery based on the first measurement, the second measurement, the first displacement, and the second displacement.

Another example provides a therapy assessment method. The method includes receiving, with a processing circuit, a stream of therapy assessment measurements obtained with a plurality of electrodes of a catheter configured to deliver PEF energy to a target tissue. The therapy assessment measurements include a first measurement taken prior to a respective time instance of PEF-energy delivery and a second measurement taken after the respective time instance. The method also includes determining, with the processing circuit, a first displacement of the plurality of electrodes with respect to the target tissue between the first measurement and the respective time instance and a second displacement of the plurality of electrodes with respect to the target tissue between the respective time instance and the second measurement. The determining operations are based on position measurements received from a tracking system configured to track a position of a selected electrode of the plurality of electrodes or of a selected tracking element. The method further includes estimating, with the processing circuit, a therapeutic effect of the PEF-energy delivery based on the first measurement, the second measurement, the first displacement, and the second displacement.

Yet another example provides a non-transitory computer-readable medium storing instructions that, when executed by an electronic processor, cause the electronic processor to perform operations comprising the above therapy assessment method.

Further disclosed herein is a medical system. The medical system includes a generator configured to generate pulsed electric field (PEF) energy. A medical device is in electrical communication with the generator and has a plurality of electrodes configured to deliver the PEF energy to a target tissue to create electroporated regions in the target tissue. A delivery element tracking system is in communication with the generator and the medical device. The tracking system has processing circuitry configured to: measure a position of at least one of the plurality of electrodes prior to delivery of PEF energy to the target tissue with respect to the target tissue and correlate a PEF field distribution based on the delivery of PEF energy to the target tissue to determine or modify at least one metric of a therapeutic effect from the PEF delivery at positions other than the measured location of the plurality of electrodes.

The details of one or more aspects of the disclosure are set forth in the accompanying drawings and the description below. Other features, objects, and advantages of the techniques described in this disclosure will be apparent from the description and drawings, and from the claims.

BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:

FIG. 1 is a system view of a PEF generator, medical devices configured to deliver PEF energy or map a tissue region, and a controller according to some examples;

FIG. 2A is a diagrammatic view of a mapped tissue region and associated PEF application points according to one example;

FIG. 2B is a diagrammatic view of one of the PEF application points shown in FIG. 2A and an electric field generated by the application of PEF energy according to one example;

FIG. 2C is the diagrammatic view shown in FIG. 2B showing multiple electric fields generated from multiple application points according to some examples;

FIG. 2D is the diagrammatic view shown in FIG. 2C showing an example display of a measure of a therapeutic effect on the tissue exposed to the applied electric field according to one example;

FIG. 3A is a diagrammatic view of a model field generated from the application of PEF energy according to one example;

FIG. 3B is a diagrammatic view of a field generated from the application of PEF energy that includes some uncertainty according to one example;

FIG. 3C is a diagrammatic view of a field generated from the application of PEF energy that includes high uncertainty according to one example;

FIG. 4 is a flow chart showing a method of determining at least one metric of a therapeutic effect from the application of PEF energy according to some examples;

FIG. 5 is a schematic diagram illustrating how motion of the heart affects the position of an electrode with respect to the mapped region of the heart during a medical procedure according to one example;

FIG. 6 is a flowchart of a method of delivering therapy to a target tissue according to some examples; and

FIG. 7 is a block diagram illustrating an example architecture of a program code that is run by the processing circuitry of the medical system of FIG. 1 to support the method of FIG. 6 according to some examples.

DETAILED DESCRIPTION

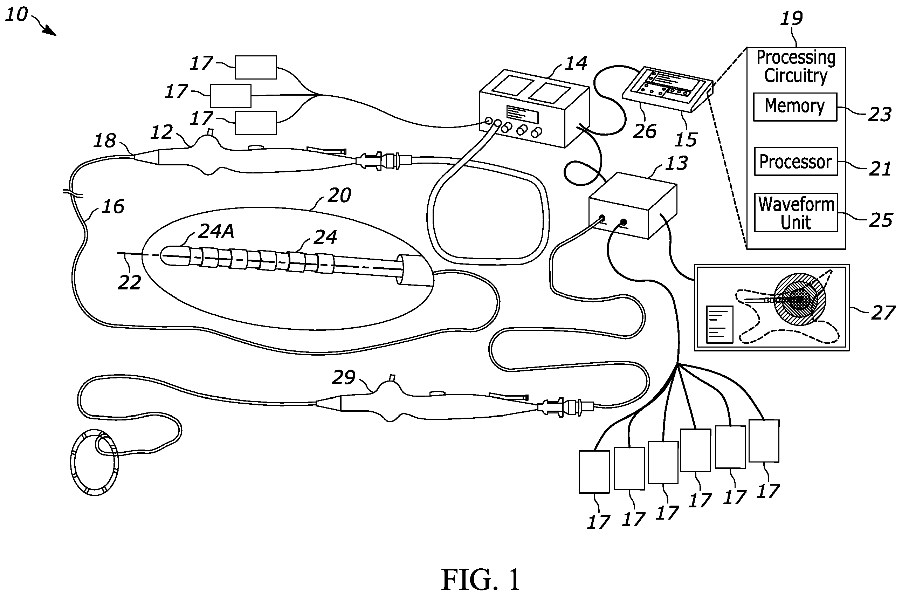

Referring now to the drawing figures in which like reference designations refer to like elements, an embodiment of a medical system constructed in accordance with principles of the present invention is shown in FIG. 1 and generally designated as "10." The system 10 generally includes a medical device 12 that may be coupled directly to an energy supply, for example, a pulse field ablation (PFA) generator 14 including an energy control, delivering, and monitoring system or indirectly through a navigation system component or delivery element tracking system 13. A remote controller 15 may further be included in communication with the generator 14 for operating and controlling the various functions of the generator 14 and in further communication with one or more surface electrodes 17 configured to measure and record electrograms. Surface electrodes 17 may also be optionally directly coupled to the generator 14 for assessment of energy application vectors. A display 27 may be in communication with the remote controller 15 and/or the navigation system component 13 to display the various maps and data generated by the various components described herein. In the example shown, the navigation system component 13 includes an impedance-based system configured to use the surface electrodes for orientation. In other examples, the navigation system component 13 may be based on other localization navigation methods, such as electromechanical or X-ray.

In one or more embodiments, the remote controller 15 may include processing circuitry 19 configured to carry out or otherwise control the various functions of the generator 14 and implement methods described herein. As shown, the processing circuitry includes a processor 21, a memory 23, and a waveform unit 25. In particular, in addition to or instead of a processor, such as a central processing unit, and memory, the processing circuitry 19 may comprise integrated circuitry for processing and/or control, e.g., one or more processors and/or processor cores and/or FPGAs (Field Programmable Gate Array) and/or ASICs (Application Specific Integrated Circuitry) adapted to execute instructions. The processor 21 may be configured to access (e.g., write to and/or read from) the memory 23, which may comprise any kind of volatile and/or nonvolatile memory, e.g., cache and/or buffer memory and/or RAM (Random Access Memory) and/or ROM (Read-Only Memory) and/or optical memory and/or EPROM (Erasable Programmable Read-Only Memory).

The processing circuitry 19 may be configured to control any of the methods and/or processes described herein and/or to cause such methods, and/or processes to be performed, e.g., via the remote controller 15. The processor 21 corresponds to one or more processors for performing functions described herein. The memory 23 is configured to store data, programmatic software code and/or other information described herein. In some embodiments, the software may include instructions that, when executed by the processor 21 and/or processing circuitry 19 causes the processor 21 and/or processing circuitry 19 to perform the processes described herein with respect to the remote controller 15. For example, processing circuitry 19 of the remote controller 15 includes the waveform unit 25 configured to perform one or more functions described herein, such as with respect to pulse generation and control.

The medical device 12 may generally include one or more diagnostic or treatment regions for energetic, therapeutic and/or investigatory interaction between the medical device 12 and a treatment site. The treatment region(s) may deliver, for example, pulsed electric field (PEF) energy sufficient to reversibly or irreversibly electroporate a tissue area, or radiofrequency (RF) energy in proximity to the treatment region(s). The medical device 12 may include an elongate body or catheter 16 passable through a patient's vasculature and/or positionable proximate to a tissue region for diagnosis or treatment, such as a catheter, sheath, or intravascular introducer. The elongate body, shaft, or catheter 16 may define a proximal portion 18 and a distal portion 20 and may further include one or more lumens disposed within the elongate body 16 thereby providing mechanical, electrical, and/or fluid communication between the proximal portion 18 of the elongate body 16 and the distal portion 20 of the elongate body 16. The distal portion 20 may generally define the one or more treatment region(s) of the medical device 12 that are operable to monitor, diagnose, and/or treat a portion of a patient. The treatment region(s) may have a variety of configurations to facilitate such operation. In the case of purely bipolar pulsed field delivery, distal portion 20 includes electrodes that form the bipolar configuration for energy delivery. A plurality of delivery elements 24, for example, a plurality of electrodes 24, may serve as one pole while a second device (e.g., second medical device 29, electrodes 17) containing one or more additional electrodes is placed to serve as the opposing pole of the bipolar configuration. The medical device 12, as shown in FIG. 1, may have a linear configuration with the plurality of delivery elements 24. For example, the distal portion 20 may include six delivery elements 24 linearly disposed along a common longitudinal axis 22. Alternatively, the distal portion 20 may include an electrode carrier arm or splines that is/are transitionable between a linear configuration and an expanded configuration in which the carrier arm or splines has an arcuate or substantially circular configuration. The carrier arm or splines may include the plurality of delivery elements 24 that are configured to deliver PEF energy. Further, the carrier arm when in the expanded configuration may lie in a plane that is substantially orthogonal to the longitudinal axis 22 of the elongate body 16. The planar orientation of the expanded carrier arm may facilitate ease of placement of the plurality of delivery elements 24 in contact with the target tissue.

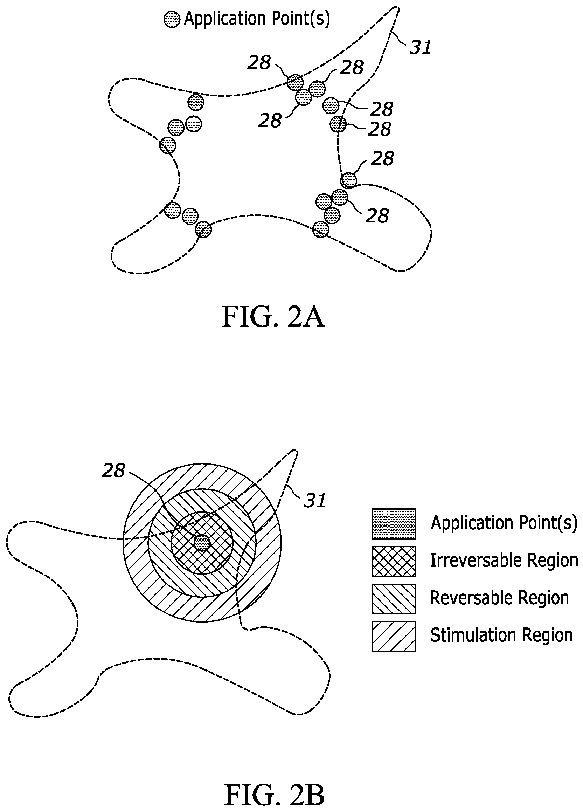

Continuing to refer to FIGS. 1-2D, the remote controller 15 further includes the delivery element tracking system 13, either integrated therein or a separate system, in communication with the generator 14 and configured to track and monitor the positions of the plurality of delivery elements 24 as the medical device 12 moves with respect to the target tissue. This communication or conveyance of information from delivery elements 24 to the navigation system occurs via the components 13 and 14, which are illustratively shown as separate systems. In operation, the components 13 and 14 make use of the connection to the delivery catheter 16, to gather additional information from the generator, e.g., for assessing cardiac cycle information for display/gating of the geometry. In some examples, the catheter 29 also communicates with the navigation system component 13. In particular, the navigation system component 13 utilizes processing circuitry 19 to measure a position of at least one of the plurality of delivery elements 24 prior to, during, and/or after generation and delivery of PEF energy to the target tissue with respect to the target tissue. For example, as shown in FIG. 2A, a plurality of PEF delivery points 28 made by repeated application of a distal tip electrode 24A is shown about a mapped region 31 of the heart. These points can be identified before, during, and after the application of PEF energy to the target region, e.g., with a second medical device 29, for example, a mapping catheter, such as the Achieve™ mapping catheter by Medtronic, Inc. The navigation system component 13 can be used to navigate to the plurality of PEF delivery points 28 and correlate a PEF field distribution based on the delivery of PEF energy to the target tissue to determine at least one metric of a therapeutic effect from the PEF delivery at positions other than the measured location of the plurality of delivery elements 24. In a representative example, an individual delivery point 28 corresponds to one PEF-energy pulse. Sequences of the individual delivery points 28 illustrated in FIG. 2A are produced when the distal tip electrode 24A is moved from one position to a next position with respect to the region 31 during the time interval between sequential PEF-energy pulses.

In one example, the determined at least one metric of a therapeutic effect is determined based at least in part on at least one from the group consisting of a temperature of the plurality of electrodes 24 during delivery of PEF energy, an impedance of the plurality of electrodes 24 during delivery of PEF energy, and a proximity of the plurality of electrodes 24 to the target tissue. Referring to FIG. 2A, in conventional ablation practice, such as with radiofrequency (RF) ablation, locations of therapeutic applications 28 are noted on maps of anatomic geometry. Placement and user selection of the icon size is meant to convey where treatment had likely happened in a typically diastolic static map and a therapeutic effect, such as ablation of a focal source or creating a line of block, when applied to treatment of atrial fibrillation, for example, as inferred from the proximity of such points and continuity of constructed lines to determine likelihood of success of the therapy. Because delivery of the RF energy generally relies on the imprecisely known or defined conduction of heat in what is an assumed homogeneous substrate (cardiomyocytes/vessel/scar/etc.), the area of effect is imprecisely characterized. In contrast, the PEF delivery points 28 in FIG. 2B can cause ablation or effect with defined field established by the electrodes 24 so the area of effects can be inferred, estimated, and/or calculated by predefined models or even empirical measurements. In one configuration, the processing circuity 19 of the controller 15 and/or delivery element tracking system 13 measures the aggregation of the intensity of the field(s) from applications of PEF energy to the delivery points 28 to determine if the geometric positions on the predetermined map of the target tissue region have been sufficiently affected to cause at least one metric of therapeutic effect, for example, irreversible electroporation.

For example, as shown in FIG. 2B, the tracking system 26 is configured to correlate the determined at least one metric of a therapeutic effect into one or more zones. For example, the zones may be visually coded (such as with at least one of a color, shading, transparency, or pattern) and displayed on the display 27 (shown in FIG. 1) overlaying the mapped target tissue region. If a shift in the map occurs, then the stored information of the applications may be reapplied to new registered geometry models. In the example shown in FIG. 2B, the pattern-coded regions or zones circumferentially disposed about a single delivery point 28 show that the farther away from the delivery point 28 the more decreased therapeutic effect from the application of the electric field.

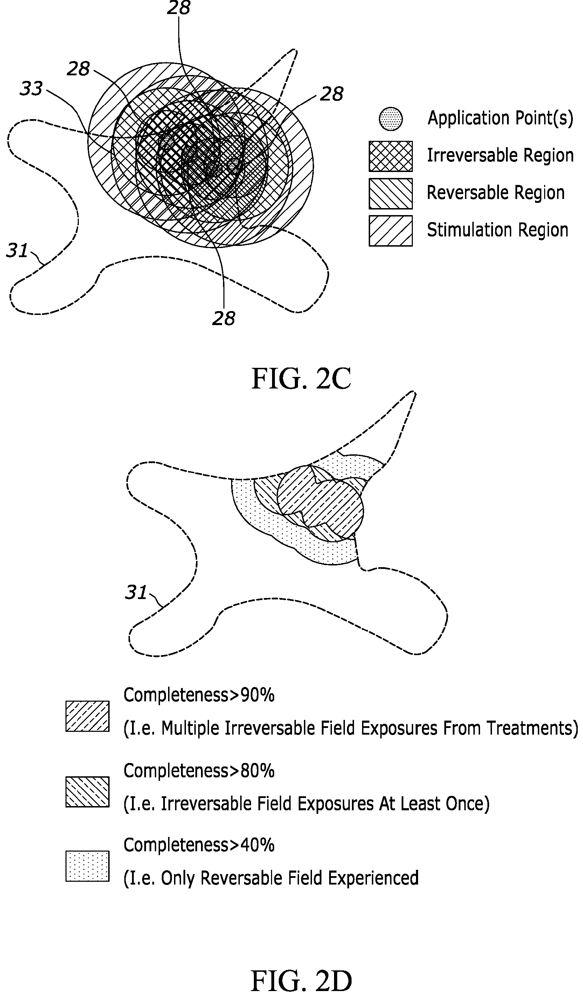

FIGS. 2C-2D show that successive applications of PEF energy, and the fidelity with which they inform the electric field that has been applied, are plotted on the map, and then the aggregated treatment of a PEF delivery point 28 may be displayed on the geometric map with some measure of completeness, such as with color/opacity. In FIG. 2C, the electroporation region 33 is shown in a bold dashed line indicating the area of tissue experiencing at least reversible electroporation. As shown in FIG. 2D, cumulative electroporation regions produced by successive applications of PEF energy to multiple delivery points 28 may have relatively complex geometries.

From the aggregated field effects of each application of PEF energy from delivery points 28, a measure of completeness of a particular energy application may be imposed on the previously generated cardiac map. In this configuration, the pattern-coded regions may correspond to a measure of completeness. For example, one region may correspond to a measure of completeness of greater than 90% if, for example, the region experiences multiple irreversible field exposures, where each point in the field may be assigned discrete count variables for such exposures in addition to any calculated values. Another region may correspond to a measure of completeness of 80% if, for example, the region experiences at least one irreversible field exposure. Yet another region may correspond to a measure of completeness of 40% if, for example, the region only experiences reversible field exposures. The above percentages are mere examples, and the field projected onto the geometric representation may change or may be modified with uncertainties coming from the location where the assessment data are collected versus where the catheter is when the field is applied. Measures of completeness can be computed incrementally and calculated in real-time. For example, a clinician can perform a set of ablations and the delivery element tracking system 26 may log and compute the field overlaps/aggregated treatments at each delivery point 28 when the map is requested by the clinician. Additionally, the measure of completeness does not have to be associated only with the geometric points, but may be done for any point within the volume being monitored by the navigation portion of the system 10, that is the points which are generally displayed as containing the heart chamber of interest for example may move, but the information about completeness may still be computed for new geometries as it is a culmination of fields associated with single placement applications. The overlap of the aggregated electro-anatomical map, i.e., containing geometry and imposed delivery data, may be predictively computed and optionally displayed for a next application. For example, as the electric field is applied around the delivery elements 24 being tracked on the map, the system 10 can show where the treated region from a next therapeutic delivery is located and/or its effect as may be added to the already collected anatomical map. This may be useful for example in helping a user determine if a placement of the delivery elements 24 is likely to close a gap in cardiac conduction or ablate another identified or unintended structure, for example. Such a prediction representation may be further modified by the PEF system 10 providing information about therapy levels, profiles, or voltages to mapping/navigation system responsible for aggregating those data and using the appropriate models.

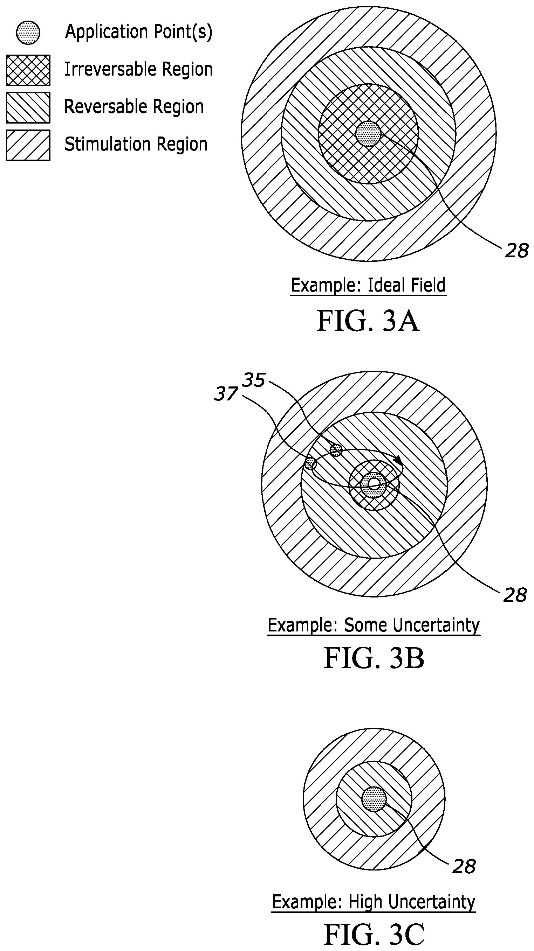

The examples of FIG. 2A-2D additionally illustrate an idealized implementation where the field is assumed to be of a consistent form and applied to the map for example as associated very specifically with a specific position and orientation of the field or zones. Variables associated directly with the delivery may be used to determine completeness and uncertainty modifications as discussed later. If pre and post measures of variables are used to determine the completeness of a delivery, the positions at which those measures are taken can additionally affect the uncertainty associated with treatment of given regions as discussed with reference to FIGS 3A-3C.

FIGS. 3A-3C show various examples of uncertainty in the field applied to a measure of completeness of particular application of field energy. When completeness is determined, at least in part, from measures such as temperature rise or impedance, for example, the real location of those measurements before and/or after a delivery can change the certainty of the relevance of those measures for assessing completeness based on their proximity to the delivery. Their proximity to one another can also be important when the difference in measurement from pre to post-delivery is important. For example, an ideal field as shown in FIG. 3A occurs when the delivery points 28 are the same points as assessment points. That is, where the field energy, i.e., current, or assessment values, are measured in the same place as where it is delivered. In such an embodiment, a measure of completeness more associated with the success of the delivery. The elements of the field shown in FIG. 3A are thus the same field regions as shown in FIG. 2B. However, as shown in FIG. 3B, when the delivery point 28 differs from the points at which the completeness/effectiveness is assessed before and after are slightly different, the field attributes are different than having been measured at the exact same location as the delivery and thus less certain to represent the success of a delivery. In the embodiment shown in FIG. 3C, the assessment points and delivery points 28 are significantly offset resulting in smaller regions of certainty for each zone.

In particular, as shown in FIG. 3B, it is possible that the point at which the field energy assessments are made after delivery, as indicated by 35 is different than the delivery point 28, or the position in which the initial position of the electrodes 24 is measured for assessment, indicated by 37. In an example application, assume that an impedance drop of at least 20% results in a 100% effective lesion. After a delivery, the impedance is measured to have dropped 20% when measured at the same point as the delivery point 28, as shown FIG. 3A. The maximum effect is applied to the model; the regions around the delivery elements 24 are computed at their maximum extent. In another similar delivery, a 16% an impedance drop is measured from the same position as the delivery points 28, and using a simple linear interpolation, this may be an 80% effective delivery. The model that is applied has reduced regions accordingly, smaller areas of effect to represent a conservative application of certainty that regions further from the delivery point(s) have been adequately treated. Yet another delivery also measures a 16% impedance drop, but the measurements are taken from positions that are different from the delivery point 28, for example, as shown in FIG. 3B. The model applied has even smaller regions of effect applied. The 80% effectiveness as given by the value of the impedance drop to be further reduced depending on how far away the values were measured from the delivery point 28 where the model may be imposed, and even from each other. Changes in the impedance may more likely represent regional differences in the heart the further away they are, reducing the effectiveness of such measures to represent the success of a delivery.

For a tracked point 28, region, zone, volume, and/or surface on a mapping system, the motion of the plurality of delivery elements 24 affects how completely a region is likely to have been treated. If a delivery element 24 is moved during progressive application of pulses in a pulse train, or successive pulse trains of PEF energy, for example, the area being treated changes and is not as effective for treating the tissue initially targeted as the area of effect is reduced by motion. For example, a time between applications in a moving heart can see seconds to minutes pass between successive applications for the same intended treatment region. Moreover, as shown in FIG. 3C, when there is high uncertainty, application points 28 are offset from assessment points owing to, for example, inaccuracies with assessments based on impedance or temperature. In such an example, the displayed field may not include an irreversible region and all the fields, for example, reversible and stimulation regions, may be reduced. In some examples, the application of the positional effect may be implemented as a standard or selectable option, allowing for higher confidence, for example, in an operator's personal assessment of their position stability in relation to the target tissue in a heart exhibiting significant motion. Therapies or entire procedures may be recalculated to the anatomical geometries based on inclusion or exclusion of such various weighting values as noted herein (e.g., one or more of position, timing, heart cycle, impedance, and temperature may be retrospectively applied or removed in practice).

Assessment fidelity is also affected by motion considerations. If a delivery element 24 provides a baseline measurement (such as impedance) in one position and then moves or shifts between that measurement and the measurement which follows, the values may be in similar locations but are less relevant, the further apart the assessment measurements are made or the further away a delivery of therapy is applied. Hence, in general form, it may be advantageous to weight measurement and therapy effectiveness measures as a function of the spatial displacements. Additional weighting may be applied to uncertainty of the position measures themselves or using the weights as measures of the uncertainty for integration with a formal filter with multiple assessment variables. Thus, when PEF energy is applied, the delivery points and the assessed position of the electrodes 24 are usually registered as separate points to reference against the geometric model to inform an operator in what vicinity a treatment has been performed. Because standard thermal techniques rely on heat transfer (such as with RF or cryo methods), treatment metrics are only associated with the position, and not generally extensible to field considerations, as suggested in the present application can be done with PEF to inform/project completeness measures to the geometric points.

A bipolar delivery represents an example implementation, wherein the field between delivery elements 24 located proximate to one another produces a more predictable field distribution as compared with a unipolar delivery. Such implementations may still be used with appropriate modeling considerations of course. It should be noted similarly that the images in FIGS. 2A-2D and 3A-3C represent such fields simplistically as point deliveries and circular fields of effects to convey the use of representative regions and implementation of uncertainty considerations. The field strength (in units of volts/cm, for example) which is an often-associated value important to the response of a particular cell type may follow the simplified geometry for regions of effect around a single conductor such as in a unipolar configuration, but the field may be far more complex between two or more delivery elements 24. The model may be computed independently for each delivery set based on individual placements of such delivery elements 24. Further, the orientation of the electric field as a vector field variable may also be incorporated into the completeness computations for a particular region as multiple orientations have been shown to be more effective at electroporating cells than with singly oriented applications (i.e., may use an angular difference in the field to assess the completeness level to which a point on the map has been treated).

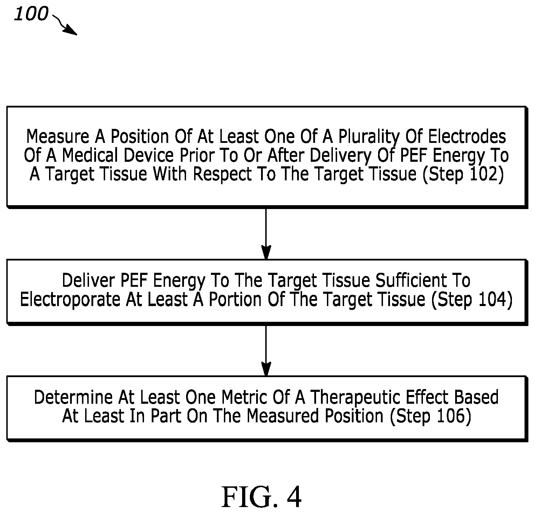

Referring now to FIG. 4, in an example method 100 of determining a region of therapeutic effect in a pulsed electric field (PEF) energy delivery system. The method 100 includes measuring a position of at least one of a plurality of electrodes 24 of a medical device 12 prior to or after delivery of PEF energy to a target tissue with respect to the target tissue, for example, a real or representative anatomical map (Step 102). For example, the position of the plurality of electrodes 24 may be determined by the delivery element tracking system 26 either before, during, or after delivery of PEF energy. The measured position may also include multiple independent measurements prior to deliveries of PEF energy taken by the delivery element tracking system 26. Moreover, the measured position may be predicted from cyclic, algorithmic, or patterned behavior in the measurements at the time of delivery of PEF energy. For example, based on the multiple independent measurements prior to the delivery, the delivery element tracking system 26 may predict the location of the plurality of electrodes 26 when PEF energy is actually delivered to the target tissue region. PEF energy is then delivered to the target tissue sufficient to electroporate at least a portion of the target tissue (Step 104). At least one metric of a therapeutic effect is determined or modified based at least in part on the measured position (Step 106). For example, based on the measured positioned of the plurality of electrodes 24 a measure of completeness may be determined and/or modified for a particular treatment region. In another words, as more information about the position of the plurality of electrodes 24 is determined, a prior measure of completeness may be updated with new measure of completeness.

Some embodiments may benefit from at least some features disclosed in U.S. Patent Application Publication No. 2022/0273353.

In some examples, a ratio of completeness C for each electrode 24 is determined based at least in part on calculating the following equation:

$$C = k \times \frac{(T_f - T_i)}{\Delta T_n} \qquad (1)$$

where k is a constant, $T_f$ is a final temperature measured at an end of a time interval, $T_i$ is the initial temperature measured at a beginning of the time interval, and $\Delta T_n$ is the expected or predicted change in temperature over that time interval. In various implementations, the expected or predicted change $\Delta T_n$ is derived from computer modeling or based on empirical observations. The expected or predicted values and model parameters for determining the expected or predicted values are stored in the memory 23.

In some other examples, the ratio of completeness C is calculated using the following equation:

$$C = k \times \frac{(Z_f - Z_i)}{\Delta Z_n} \qquad (2)$$

where k is a constant, $Z_f$ is the final impedance at the end of a time interval, $Z_i$ is the initial impedance at the beginning of the time interval, and $\Delta Z_n$ is the expected or predicted change in impedance over that time interval. In various implementations, the impedance values may be complex or real. In some cases of complex impedance values, the completeness may first be computed as a complex value, which may then be reduced to a real value for ease of understanding by the users. In various implementations, the expected or predicted change $\Delta Z_n$ is derived from computer modeling or based on empirical observations. In yet some other examples, voltage-, current-, and EGM-based calculations of the ratio of completeness C are implemented in the same or substantially similar manner.

In additional examples, a cumulative ratio of completeness $C_{total}$ is calculated using the following equation:

$$C_{total} = \sum_i \omega_i C_i \qquad (3)$$

where $i$ is a summation index, $\omega_i$ is the i-th weight, and $C_i$ is the i-th individual ratio of completeness. In various examples, different values of the index $i$ correspond to different time instances, different sensors, different electrode configurations, different displacement values, etc. In some of such examples, at least some of the i-th ratios $C_i$ are determined in accordance with Eq. (1). In other ones of such examples, at least some of the i-th ratios $C_i$ are determined in accordance with Eq. (2). In yet other ones of such examples, at least some of the i-th ratios $C_i$ are determined based on the voltage-, current-, and/or EGM-based measurements and calculations.

In general, calculations in accordance with Eq. (3) can be performed based on measurements performed by any suitable set of sensors available for such measurements in the medical devices 12 and/or 29. In some instances, the available set of sensors consists of sensors of a single type, such as temperature sensors or impedance sensors. In some other instances, the available set of sensors includes sensors of two or more different types, such as temperature sensors, impedance sensors, and so on. In various examples, some or all of the sensors are implemented using the electrodes 24. For example, in some embodiments, a temperature sensor is implemented using copper and constantan wire leads connected to an electrode 24 to form an end of a thermocouple thereat. In various embodiments, sensing of the voltage and/or current between a selected pair of electrodes 24 is used for impedance measurements.

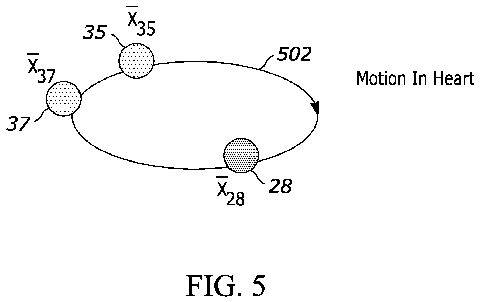

FIG. 5 is a schematic diagram illustrating how cyclic motion of the heart affects or may be used to calculate or infer the position of an electrode 24 with respect to the mapped region 31 of the heart during a medical procedure according to one example. Since motion of the heart is generally cyclical, such motion is represented in FIG. 5 by a looped trajectory 502. A PEF energy pulse is delivered when the corresponding electrode 24 is at the application point 28, e.g., as explained above in reference to FIGS. 2A-2D and 3A-3C. Even when the electrode 24 is stationary in the tissue coordinate frame, the electrode 24 still moves with respect to the mapped region 31 along the looped trajectory 502 due to the motion of the heart. In some examples, the electrode(s) 24 may be in contact with the target tissue, and it is the motion of that tissue which presents itself as the motion relative to a static displayed anatomy. In some other examples, it may be the case that the cyclic motion not adjacent to the target tissue still induces motion of the electrode(s) 24 relative to the target tissue. As such, assessment measurements taken using the same electrode 24 may occur at locations that are different from the application point 28, depending on the specific phase of the cardiac cycle. As an example, FIG. 5 illustrates three different points 28, 35, and 37 on the looped trajectory 502 for the same electrode 24 during different time instances of the medical procedure. More specifically, the point 37 marks the position of the electrode 24 at which the initial (baseline) measurement of the pertinent parameter (e.g., temperature or impedance) is taken. Similarly, the point 35 marks the position of the electrode 24 at which the post-delivery measurement of the pertinent parameter is taken. Both of the points 35 and 37 correspond to different respective phases of the cardiac cycle, which are also different from the phase corresponding to the application point 28. This relative phase, such as a percentage estimation of the cardiac cycle based on electrocardiogram measures of the R-R interval may itself be incorporated into the timing and representation of the applied field and calculation of the completeness as it may be the case that different phases of contraction have a different response to therapy (e.g., the stimulation range of an electric field may have no value during a local refractory period).

In general, for a tracked point on a mapping system, the motion of the delivery elements, such as that of the electrodes 24, affects how completely the mapped region 31 is treated. When an electrode 24 is moved during progressive application of pulses in a pulse train or successive pulse trains, the area of the mapped region 31 that is being treated changes accordingly. In some examples, the corresponding electrode movement includes a non-zero displacement component in the laboratory coordinate frame in addition to the movement component along the looped trajectory 502. Due to these movements, the treatment effect may not be nearly the same as intended because such treatment effect is spatially dispersed by the above-indicated motion. However, corrective measures can beneficially be taken provided that the cumulative ratio of completeness $C_{total}$ can be accurately estimated.

In various examples, assessment fidelity is also affected by similar motion considerations. For example, when an electrode 24 provides a baseline measurement (such as an impedance measurement or a temperature measurement) in the point 37 and then moves or shifts to the point 35 for the next measurement, the measured values are typically less relevant to the mapping than those putatively obtained at the application point 28. In general, the relevance is diminished when the assessment measurements are made further apart or when the points 35, 37 are further away from the application point 28. The weights $\omega_i$ (Eq. (3)) are therefore used to introduce an appropriate spatial-displacement dependence into the assessment of therapy effectiveness. In some implementations, additional weighting is applied to take into account the uncertainty in the measures of the positions 28, 35, and/or 37 themselves or to use the weights as measures of the uncertainty for integration with a filter configured for multiple assessment variables. Examples of such uncertainty are illustrated in FIGS. 3A-3C.

In general, the weights associated with the position are a function of the spatial positions for each of the relevant measures, e.g., as expressed by Eq. (4):

$$\omega_i = F(\boldsymbol{X}_{28}, \boldsymbol{X}_{35}, \boldsymbol{X}_{37}) \qquad (4)$$

where F denotes a function; and $\boldsymbol{X}_{28}$, $\boldsymbol{X}_{35}$, and $\boldsymbol{X}_{37}$ are the coordinates (in vector form) of the points 28, 35, and 37, respectively. Eqs. (5)-(6) are examples of the function F according to some implementations:

$$\omega_i = \frac{a}{|\boldsymbol{X}_{37} - \boldsymbol{X}_{35}|} \qquad (5)$$

$$\omega_i = \frac{b}{|(\boldsymbol{X}_{37} + \boldsymbol{X}_{35})/2 - \boldsymbol{X}_{35}|} \qquad (6)$$

where $a$ and $b$ are constants. Other suitable implementations of the function F are also possible.

In the above examples, the vector $\boldsymbol{X}$ is a position vector that represents geometric coordinates. In additional examples, the vector $\boldsymbol{X}$ is generalized to include a time component and/or a phase component. Eqs. (7)-(10) provide mathematical expressions for different implementations of the vectors $\boldsymbol{X}$ to be used with the function F of Eq. (4):

$$\boldsymbol{X} = (x, y, z) \qquad (7)$$

$$\boldsymbol{X} = (x, y, z, t) \qquad (8)$$

$$\boldsymbol{X} = (x, y, z, \phi) \qquad (9)$$

$$\boldsymbol{X} = (x, y, z, t, \phi) \qquad (10)$$

where $x, y, z$ are the cartesian spatial coordinates; $t$ is time; and $\phi$ is the phase of the cardiac cycle. In some examples, the phase $\phi$ is determined as follows:

$$\phi = \frac{2\pi T}{T_{r2} - T_{r1}} \qquad (11)$$

where $T_{r2}$ and $T_{r1}$ are the times of two consecutive R-waves, and $T$ is a time between the times $T_{r2}$ and $T_{r1}$.

The spatial coordinates are important for the determination of the weights $\omega_i$ for the above-indicated reasons. In some use cases, the time component is important to the physiological response. In some use cases, the phase component or an estimate thereof is important for correlation of the cyclic motion in the heart along the looped trajectory 502 with the cardiac cycle. For example, when an electrode 24 takes an assessment measure at one time and the spatial position is the same as at another time, but at a different phase of the cardiac cycle, it might not assertively be assumed that the same cardiac tissue is in proximity to the electrode 24 delivering the therapy. It should also be noted that increased permeabilization is observed to persist in cells only for a limited period of time after the PEF application when the cell is "reversibly" electroporated. In such cases, the cell will eventually recover, and the permeabilization will decrease over time as the cell progresses towards full recovery. However, this increased-permeabilization state makes it more likely for the cell to transition into being irreversibly electroporated with subsequent PEF-energy applications (e.g., pulse trains delivered on the order of seconds to minutes after the previous application) while the cell is still in the increased-permeabilization state. The time periods associated with the permeabilization are tissue dependent as well and may serve as model inputs. In a single variable application, the time variable may be used for mapping the rate of change of measured values, such as impedance, with multiple measures of the variable after a delivery to determine derivative values which may themselves be useful for assessing success/effect of a therapy.

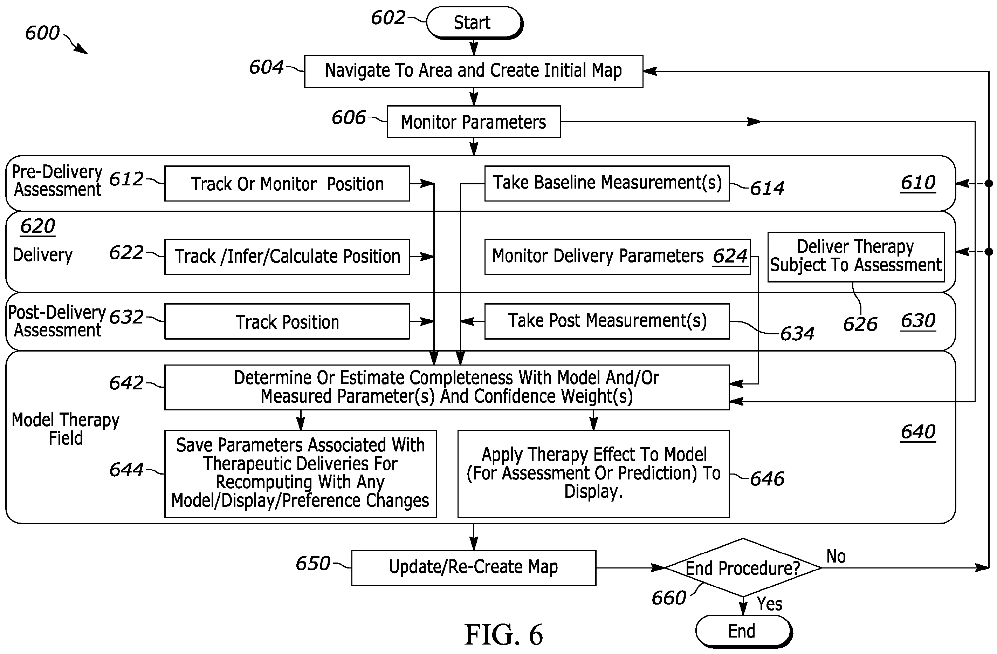

FIG. 6 is a flowchart of a method 600 of delivering therapy to a target tissue according to some examples. The method 600 can be implemented using the medical system 10 (FIG. 1) and includes operations directed at assessing completeness of the delivered therapy. The completeness-assessment operations of the method 600 take into account the above-described spatial dispersion of the delivered PEF energy and the relative displacements of the application point(s) 28 and assessment points 35, 37.

Initialization operations of the method 600 include operations of blocks 602, 604, and 606. In a representative example, initialization operations of the block 602 include some or all of the following: (i) overall system setup; (ii) initialization of the catheter navigation subsystem of the system 10; (iii) initialization of the PFA generator 14; (iv) loading up a model of the selected therapy into the memory 23 and/or the processor 21; (v) identifying and/or defining the target tissue; (vi) loading the geometry of the distal portion 20 of the catheter into the model and selecting delivery elements therein; (vii) creating or importing the initial map of the therapy-delivery area and loading the initial map into the memory 23 and/or the processor 21, and the like. Operations of the block 604 include initializing a navigation subsystem (e.g., including element tracking system 26 or similar) of the medical system 10 and navigating the medical device(s) 12 and/or 29 through the vasculature to the therapy-delivery area using the navigation subsystem which may include creation of the initial mapping geometry (initial map). Operations of the block 606 include initializing a heart monitoring subsystem of the medical system 10 to start monitoring the patient's cardiac cycle. In various examples, such initializing involves appropriately positioning the one or more surface electrodes 17 on the skin of the patient and activating the signal recorder to record, e.g., the electrocardiogram (ECG) R-wave detection history. Depending on the specific embodiment of the medical system 10, blocks 602, 604, and 606 may include additional appropriate initialization operations known to persons of ordinary skill in the pertinent art.

Upon completion of the above-described initialization operations, the method 600 proceeds to perform operations of blocks 610, 620, 630, and 640. The block 610 includes operations directed at pre-delivery assessment of the therapy-delivery area using baseline measurements performed in a sub-block 614 thereof. The block 620 includes delivering the PEF energy to the target tissue in a sub-block 626 with the actual PEF-energy delivery parameters being monitored in a sub-block 624. The block 630 includes operations directed at post-delivery assessment of the therapy-delivery area using post-delivery measurements performed in a sub-block 634 thereof. The blocks 610, 620, 630 also include respective medical-device tracking operations 612, 622, 632, wherein the navigation subsystem and/or other appropriate means are used to obtain pertinent values of the vectors $\mathbf{X}_{28}$, $\mathbf{X}_{35}$, and $\mathbf{X}_{37}$. For example, in some cases, the position tracking measurements in the block 622 are supplemented by calculations or indirect inferences to more-precisely pinpoint the location $\mathbf{X}_{28}$ of the delivery element (e.g., the active electrode 24) with respect to the region 31 during the actual (short) instance of the corresponding PEF pulse. Such calculations or inferences may not be needed for the sub-blocks 612 and/or 632 as the measurements 614, 634 are performed on a different time scale than that of the PEF delivery of the sub-block 626.

The block 640 includes operations 642 directed at determining or estimating a completeness measure of the therapy that is being applied. Numerical inputs for the operations 642 are provided by the position-tracking data obtained in the sub-blocks 612, 622, and 632, the measurements (of temperature, impedance, etc.) taken in the sub-blocks 614 and 634, the PEF-energy delivery parameters obtained in the sub-block 624, and the cardiac-cycle data obtained via the block 606. In various examples, the position-tracking data obtained in the sub-blocks 612, 622, and 632 provide the *x, y, z* components of the vectors $\mathbf{X}_{28}$, $\mathbf{X}_{35}$, and $\mathbf{X}_{37}$. The cardiac-cycle data obtained in the block 606 are used to determine the *t* and/or $\phi$ components of the vectors $\mathbf{X}_{28}$, $\mathbf{X}_{35}$, and $\mathbf{X}_{37}$ (also see Eqs. (7)-(10)). The *t* component, as described above, may also depend on the time of delivery from previous deliveries (also see the block 620. The operations 642 include a set of operations directed at computing the weights $\omega_i$ based on the received numerical inputs and using the applicable form of the function F. In various examples, Eqs. (4)-(6) or functionally similar numerical or analytical models are used for such computations. The operations 642 further include a set of operations directed at computing measures of completeness based on the measurements obtained in the sub-blocks 614, 634 and further based on the computed weights $\omega_i$. In various examples, Eqs. (1)-(3) or functionally similar numerical or analytical models are used for computing the measures of completeness. The block 640 also includes operations 644 directed at saving the parameters associated with the therapeutic deliveries for recomputing with any future model/display/preference changes. The block 640 further includes operations 646 directed at applying the estimated therapy effect to the applicable model of the treatment area, e.g., for assessment or prediction, and displaying the results to the operator. Examples of the estimated therapy effect for individual application points 28 are described above, e.g., in reference to FIGS. 2B and 3A-3C.

A block 650 of the method 600 includes operations directed at generating a treatment completeness map that reflects all previous PEF-energy applications to the treatment area based on the computations performed in the preceding instances of the block 640 for various application points 28. An example of such treatment completeness map is shown in FIG. 2D. An example of how the computations corresponding to individual application points 28 are combined to generate the treatment completeness map in the block 650 are illustrated in FIG. 2C.

A decision block 660 of the method 600 is used to determine whether to end the treatment. The decision (Yes or No) made in the decision block 660 is based on the treatment completeness map generated in the block 650. Upon sufficient completeness (which is determined based on the applicable medical criteria for the type of treatment that is being performed) indicated by the treatment completeness map ("Yes" at the decision block 660), the method 600 is terminated. Otherwise ("No" at the decision block 660), the processing of the method 600 is directed back to the operations of the block 604, wherein position of the treatment elements is adjusted by navigating the same to the area of the target tissue that is indicated as being insufficiently treated in the treatment completeness map for additional PEF-energy applications. In various embodiments, the method 600 may be adapted to performing a local treatment, e.g., directed at isolating a vessel, or to performing a more comprehensive treatment as a complete therapeutic case, wherein multiple (e.g., disjoint) target areas are treated. In some examples associated with such embodiments, the "No" decision at the block 660 directs the processing of the method 600 to the block 610 or 620, rather than to the block 604.

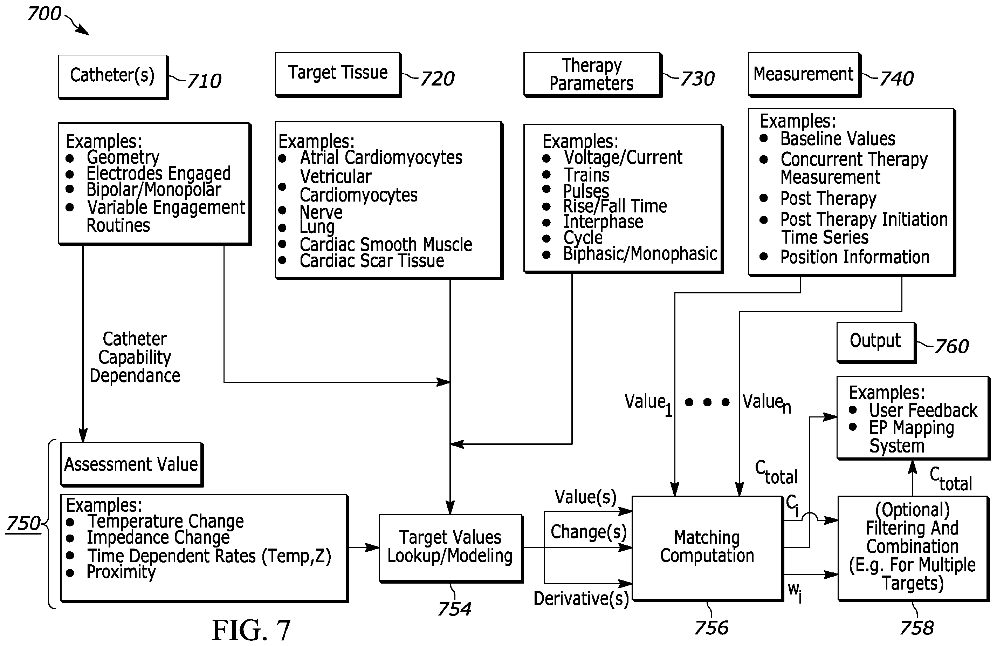

FIG. 7 is a block diagram illustrating a program code (software) 700, e.g., run by the processing circuitry 19, to support the method 600 according to some examples. The program code 700 has several modules configured to handle different respective aspects of the method 600. For example, a catheter module 710 is used to handle the catheter-related aspects, which include but are not limited to: (i) geometry (e.g., linear, circular, splined, or adjustable) of the distal portion (e.g., 20, FIG. 1) of the corresponding medical device; (ii) selection of the electrodes 24 for PEF-energy delivery and assessment measurements; (iii) geometric modality of the PEF-energy delivery (e.g., bipolar or unipolar); and (iv) sequences in which the selected electrodes 24 are engaged for various energy-delivery and assessment operations. A target-tissue module 720 is used to handle the target-tissue related aspects, as the same medical device can be used for treating different target tissues. Examples of the target tissues covered by target-tissue module 720 include but are not limited to atrial cardiomyocytes, ventricular cardiomyocytes, nerve, lung, cardiac smooth muscle, and cardiac scar tissue. In various examples, the target-tissue module 720 provides pertinent physiological models for each target tissue selection. An therapy-parameters module 730 is used to handle the energy-level aspects, as the same catheter can be used for delivering different PEF waveforms, and the catheter module 710 may inform the module 730 as to what the catheter is capable of delivering. Examples of waveform parameters covered by the module 730 include amplitudes of voltages and/or currents, pulse train parameters, individual pulse parameters, and various waveform-shape parameters, such as rise and fall times, interphase delays, cycle times, and voltage/current polarity (e.g., biphasic or monophasic). A measurement module 740 is used to handle the measurement aspects of the therapy delivery. Examples of the measurements handled via the measurement module 740 include but are not limited to: (i) tracking spatial, time, and phase components of the various pertinent vectors $\mathbf{X}$; (ii) baseline values, e.g., obtained via the sub-block 614 of the method 600; (iii) actual PEF-energy delivery parameters, e.g., obtained via the sub-block 624 of the method 600; and (iv) post-delivery values, e.g., obtained via the sub-block 634 of the method 600.

The modules 710, 720, 730, and 740 feed the respective data sets into a computation pipeline 750 that is configurable for a variety of selected assessment values, such as temperature change, impedance change, time-dependent rates of the temperature and/or impedance changes, etc. A target computation module 754 of the pipeline 750 uses the data sets provided by the modules 710, 720, and 730 to compute various target values for the given catheter geometry, target tissue, and selected PEF-energy modality. The computed target values are then used as references against which the mapped completeness values $C_{total}$ can be evaluated. In various examples, the mapped completeness

values $C_{total}$ are computed, via a matching computation module 756 and an optional filtering and combination module 758 of the pipeline 750, based on the measurements Value$_l$-Value$_n$ received via the measurement module 740. The computations performed in the module 756 typically include computations of the weights $\omega_i$ and individual completeness ratios $C_i$. When the optional filtering and combination operations of the module 758 are bypassed, the module 756 operates to compute the mapped completeness values $C_{total}$ therein. In cases of multiple targets, the module 758 receives the weights $\omega_i$ and completeness ratios $C_i$ from the module 756 and operates to compute the mapped completeness values $C_{total}$ after applying the pertinent filtering and combination operations therein.

An output module 760 receives the mapped completeness values $C_{total}$ from the pipeline 750 for presentation to the operator and/or further evaluation. In one example, the output module 760 outputs a completeness map of the region 31 that is qualitatively similar to the completeness map shown in FIG. 2D. In some examples, the output module 760 enables the operator to input the user feedback into the completeness map, e.g., for taking responsive corrective actions via the return path 660→604 of the method 600. In some other examples, the output module 760 presents a predicted image alone or in combination with the map of therapy already applied given the settings, position, and set, default, or estimated values for the therapy completeness.

According to one example disclosed above, e.g, in the summary section and/or in reference to any one or any combination of some or all of FIGS. 1-7, provided is a medical treatment apparatus, comprising: a catheter having a plurality of electrodes configured to deliver PEF energy to a target tissue and further configured for therapy assessment measurements in the target tissue, the therapy assessment measurements including a first measurement taken prior to a respective time instance of PEF-energy delivery and a second measurement taken after the respective time instance; a tracking system configured to track a position of a selected electrode of the plurality of electrodes to determine a first displacement of the plurality of electrodes with respect to the target tissue between the first measurement and the respective time instance and a second displacement of the plurality of electrodes with respect to the target tissue between the respective time instance and the second measurement; and a processing circuit configured to estimate a therapeutic effect of the PEF-energy delivery based on the first measurement, the second measurement, the first displacement, and the second displacement.

In some examples of the above medical treatment apparatus, the first measurement includes a first temperature measurement using a first electrode of the plurality of electrodes and a second temperature measurement using a second electrode of the plurality of electrodes; and wherein the second measurement includes a third temperature measurement using the first electrode and a fourth temperature measurement using the second electrode.

In some examples of any of the above medical treatment apparatus, the first measurement includes a first impedance measurement using a first electrode pair of the plurality of electrodes and a second impedance measurement using a second electrode pair of the plurality of electrodes; and wherein the second measurement includes a third impedance measurement using the first electrode pair and a fourth impedance measurement using the second electrode pair.

In some examples of any of the above medical treatment apparatus, the first measurement includes a first temperature measurement and a first impedance measurement; and wherein the second measurement includes a second temperature measurement and a second impedance measurement.

In some examples of any of the above medical treatment apparatus, the medical treatment apparatus further comprises a cardiac-cycle monitor, wherein the processing circuit is further configured to estimate the therapeutic effect based on one or more cardiac-cycle phases selected from the group consisting of: a first cardiac-cycle phase corresponding to the first measurement; a second cardiac-cycle phase corresponding to the time instant; and a third cardiac-cycle phase corresponding to the second measurement.

In some examples of any of the above medical treatment apparatus, the medical treatment apparatus further comprises a waveform generator in electrical communication with the plurality of electrodes to apply thereto a sequence of pulses of the PEF energy, wherein the processing circuit is further configured to estimate the therapeutic effect based on a time delay between a pair of consecutive pulses of the PEF energy in the sequence of pulses.

In some examples of any of the above medical treatment apparatus, the processing circuit is further configured to estimate the therapeutic effect based on a cumulative measure of completeness computed as a weighted sum of a plurality of different individual measures of completeness.

In some examples of any of the above medical treatment apparatus, the plurality of different individual measures of completeness includes two or more individual measures of completeness selected from the group consisting of: a pair of individual measures of completeness corresponding to different respective time instances of the PEF-energy delivery; a pair of individual measures of completeness corresponding to different respective types of measured values (e.g., temperature and impedance); a pair of individual measures of completeness corresponding to different respective subsets of the plurality of electrodes; and a pair of individual measures of completeness corresponding to different respective sets of displacement values.

In some examples of any of the above medical treatment apparatus, the processing circuit includes circuitry configured to generate a zoned PEF-energy effect map for an individual PEF-energy application point corresponding to the respective time instance based on the first measurement, the second measurement, the first displacement, and the second displacement.

In some examples of any of the above medical treatment apparatus, the circuitry is configured to generate a treatment completeness map by combining a plurality of zoned PEF-energy effect maps corresponding to different individual PEF-energy application points.

According to another example disclosed above, e.g., in the summary section and/or in reference to any one or any combination of some or all of FIGS. 1-7, provided is a therapy assessment method, comprising: receiving, with a processing circuit, a stream of therapy assessment measurements obtained with a plurality of electrodes of a catheter configured to deliver pulsed electric field (PEF) energy to a target tissue, the therapy assessment measurements including a first measurement taken prior to a respective time instance of PEF-energy delivery and a second measurement taken after the respective time instance; determining, with the processing circuit, a first displacement of the plurality of electrodes with respect to the target tissue between the first measurement and the respective time instance and a second displacement of the plurality of electrodes with respect to the target tissue between the respective time instance and the second measurement, the determining being based on position measurements received from a tracking system configured to track a position of a selected electrode of the plurality of electrodes; and estimating, with the processing circuit, a therapeutic effect of the PEF-energy delivery based on the first measurement, the second measurement, the first displacement, and the second displacement.

In some examples of the above therapy assessment method, the estimating is further based on one or more cardiac-cycle phases selected from the group consisting of: a first cardiac-cycle phase corresponding to the first measurement; a second cardiac-cycle phase corresponding to the time instant; and a third cardiac-cycle phase corresponding to the second measurement

In some examples of any of the above therapy assessment methods, the estimating is further based on a time delay between a pair of consecutive pulses of the PEF energy in a sequence of pulses applied to the plurality of electrodes by a waveform generator.

In some examples of any of the above therapy assessment methods, the estimating is further based on a cumulative measure of completeness computed as a weighted sum of a plurality of different individual measures of completeness

In some examples of any of the above therapy assessment methods, the estimating is further based on a cumulative measure of completeness computed as a weighted sum of a plurality of different individual measures of completeness.

In some examples of any of the above therapy assessment methods, the plurality of different individual measures of completeness includes two or more individual measures of completeness selected from the group consisting of: a pair of individual measures of completeness corresponding to different respective time instances of the PEF-energy delivery; a pair of individual measures of completeness corresponding to different respective types of measured values; a pair of individual measures of completeness corresponding to different respective subsets of the plurality of electrodes; and a pair of individual measures of completeness corresponding to different respective sets of displacement values.

In some examples of any of the above therapy assessment methods, the method further comprises generating, with the processing circuit, a zoned PEF-energy effect map for an individual PEF-energy application point corresponding to the respective time instance based on the first measurement, the second measurement, the first displacement, and the second displacement.

In some examples of any of the above therapy assessment methods, the method further comprises generating, with the processing circuit, a treatment completeness map by combining a plurality of zoned PEF-energy effect maps corresponding to different individual PEF-energy application points.

According to yet another example disclosed above, e.g., in the summary section and/or in reference to any one or any combination of some or all of FIGS. 1-7, provided is a non-transitory computer-readable medium storing instructions that, when executed by an electronic processor, cause the electronic processor to perform operations comprising any one of the above therapy assessment methods.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g, RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

It will be appreciated by persons skilled in the art that the present invention is not limited to what has been particularly shown and described herein above. In addition, unless mention was made above to the contrary, it should be noted that all of the accompanying drawings are not to scale. A variety of modifications and variations are possible in light of the above teachings without departing from the scope of the invention, which is limited only by the following claims.

**Claims**

1.  A medical treatment apparatus, comprising:

    a catheter having a plurality of electrodes configured to deliver pulsed electric field (PEF) energy to a target tissue and further configured for taking therapy assessment measurements in the target tissue including a first measurement and a second measurement;
    a tracking system configured to track a position of a selected electrode of the plurality of electrodes or of a selected tracking element to determine a first displacement of the plurality of electrodes between the first measurement and a respective time instance of PEF-energy delivery and a second displacement of the plurality of electrodes with respect to the target tissue between the respective time instance and the second measurement; and
    a processing circuit configured to estimate a therapeutic effect of the PEF-energy delivery based on the first measurement, the second measurement, the first displacement, and the second displacement,
    wherein the first measurement is taken prior to the respective time instance; and
    wherein the second measurement is taken after the respective time instance.

2.  The medical treatment apparatus of claim 1,

    wherein the first measurement includes a first temperature measurement using a first electrode of the plurality of electrodes and a second temperature measurement using a second electrode of the plurality of electrodes; and
    wherein the second measurement includes a third temperature measurement using the first electrode and a fourth temperature measurement using the second electrode.

3.  The medical treatment apparatus of claim 1 or 2,

    wherein the first measurement includes a first impedance measurement using a first electrode pair of the plurality of electrodes and a second impedance measurement using a second electrode pair of the plurality of electrodes; and
    wherein the second measurement includes a third impedance measurement using the first electrode pair and a fourth impedance measurement using the second electrode pair.

4.  The medical treatment apparatus of any of the claims 1 to 3,

    wherein the first measurement includes a first temperature measurement and a first impedance measurement; and
    wherein the second measurement includes a second temperature measurement and a second impedance measurement.

5.  The medical treatment apparatus of any of the claims 1 to 4, further comprising a cardiac-cycle monitor,
    wherein the processing circuit is further configured to estimate the therapeutic effect based on one or more cardiac-cycle phases selected from the group consisting of:

    a first cardiac-cycle phase corresponding to the first measurement;
    a second cardiac-cycle phase corresponding to the respective time instant; and
    a third cardiac-cycle phase corresponding to the second measurement.

**6.** The medical treatment apparatus of any of the claims 1 to 5, further comprising a waveform generator in electrical communication with the plurality of electrodes to apply thereto a sequence of pulses of the PEF energy, wherein the processing circuit is further configured to estimate the therapeutic effect based on a time delay between a pair of consecutive pulses of the PEF energy in the sequence of pulses.

**7.** The medical treatment apparatus of any of the claims 1 to 6, wherein the processing circuit is further configured to estimate the therapeutic effect based on a cumulative measure of completeness computed as a weighted sum of a plurality of different individual measures of completeness, particularly wherein the plurality of different individual measures of completeness includes two or more individual measures of completeness selected from the group consisting of:

a pair of individual measures of completeness corresponding to different respective time instances of the PEF-energy delivery;
a pair of individual measures of completeness corresponding to different respective types of measured values;
a pair of individual measures of completeness corresponding to different respective subsets of the plurality of electrodes; and
a pair of individual measures of completeness corresponding to different respective sets of displacement values.

**8.** The medical treatment apparatus of any of the claims 1 to 7, wherein the processing circuit includes circuitry configured to generate a zoned PEF-energy effect map for an individual PEF-energy application point corresponding to the respective time instance based on the first measurement, the second measurement, the first displacement, and the second displacement, particularly wherein the circuitry is configured to generate a treatment completeness map by combining a plurality of zoned PEF-energy effect maps corresponding to different individual PEF-energy application points.

**9.** A therapy assessment method, comprising:

receiving, with a processing circuit, a stream of therapy assessment measurements obtained with a plurality of electrodes of a catheter configured to deliver pulsed electric field (PEF) energy to a target tissue, the therapy assessment measurements including a first measurement taken prior to a respective time instance of PEF-energy delivery and a second measurement taken after the respective time instance;
determining, with the processing circuit, a first displacement of the plurality of electrodes between the first measurement and the respective time instance and a second displacement of the plurality of electrodes between the respective time instance and the second measurement, the determining being based on position measurements received from a tracking system configured to track a position of a selected electrode of the plurality of electrodes or of a selected tracking element; and
estimating, with the processing circuit, a therapeutic effect of the PEF-energy delivery based on the first measurement, the second measurement, the first displacement, and the second displacement.

**10.** The therapy assessment method of claim 9, wherein the estimating is further based on one or more cardiac-cycle phases selected from the group consisting of:

a first cardiac-cycle phase corresponding to the first measurement;
a second cardiac-cycle phase corresponding to the respective time instant; and
a third cardiac-cycle phase corresponding to the second measurement.

**11.** The therapy assessment method of claim 9 or 10, wherein the estimating is further based on a time delay between a pair of consecutive pulses of the PEF energy in a sequence of pulses applied to the plurality of electrodes by a waveform generator, and/or wherein the estimating is further based on a cumulative measure of completeness computed as a weighted sum of a plurality of different individual measures of completeness.

**12.** The therapy assessment method of any of the claims 9 to 11, wherein the estimating is further based on a time delay between a pair of consecutive pulses of the PEF energy in a sequence of pulses applied to the plurality of electrodes by a waveform generator.

**13.** The therapy assessment method of any of the claims 9 to 12, wherein the estimating is further based on a cumulative measure of completeness computed as a weighted sum of a plurality of different individual measures of completeness, particularly
wherein the plurality of different individual measures of completeness includes two or more individual measures of completeness selected from the group consisting of:

a pair of individual measures of completeness corresponding to different respective time instances of the PEF-energy delivery;
a pair of individual measures of completeness corresponding to different respective types of measured values;
a pair of individual measures of completeness corresponding to different respective subsets of the plurality of electrodes; and
a pair of individual measures of completeness corresponding to different respective sets of displacement values.

14. The therapy assessment method of any of the claims 9 to 13, further comprising generating, with the processing circuit, a zoned PEF-energy effect map for an individual PEF-energy application point corresponding to the respective time instance based on the first measurement, the second measurement, the first displacement, and the second displacement, particularly
further comprising generating, with the processing circuit, a treatment completeness map by combining a plurality of zoned PEF-energy effect maps corresponding to different individual PEF-energy application points.

15. A non-transitory computer-readable medium storing instructions that, when executed by an electronic processor, cause the electronic processor to perform operations comprising the method of any of the claims 9 to 14.

**Patentansprüche**

1. Medizinische Behandlungsvorrichtung, umfassend:

einen Katheter, der mehrere Elektroden aufweist, die dazu ausgelegt sind, Energie eines gepulsten elektrischen Feldes (PEF) an ein Zielgewebe abzugeben und ferner dazu ausgelegt, Therapiebeurteilungsmessungen in dem Zielgewebe vorzunehmen, die eine erste Messung und eine zweite Messung beinhalten;
ein Verfolgungssystem, das dazu ausgelegt ist, eine Position einer ausgewählten Elektrode der mehreren Elektroden oder eines ausgewählten Verfolgungselements zu verfolgen, um eine erste Verschiebung der mehreren Elektroden zwischen der ersten Messung und einer jeweiligen Zeitinstanz einer PEF-Energieabgabe und einer zweiten Verschiebung der mehreren Elektroden im Hinblick auf das Zielgewebe zwischen der jeweiligen Zeitinstanz und der zweiten Messung zu bestimmen; und
eine Verarbeitungsschaltung, die dazu ausgelegt ist, eine therapeutische Wirkung der PEF-Energieabgabe basierend auf der ersten Messung, der zweiten Messung, der ersten Verschiebung und der zweiten Verschiebung zu schätzen, wobei die erste Messung vor der jeweiligen Zeitinstanz vorgenommen wird; und
wobei die zweite Messung nach der jeweiligen Zeitinstanz vorgenommen wird.

2. Medizinische Behandlungsvorrichtung nach Anspruch 1,

wobei die erste Messung eine erste Temperaturmessung unter Verwendung einer ersten Elektrode der mehreren Elektroden und eine zweite Temperaturmessung unter Verwendung einer zweiten Elektrode der mehreren Elektroden beinhaltet; und
wobei die zweite Messung eine dritte Temperaturmessung unter Verwendung der ersten Elektrode und eine vierte Temperaturmessung unter Verwendung der zweiten Elektrode beinhaltet.

3. Medizinische Behandlungsvorrichtung nach Anspruch 1 oder 2,

wobei die erste Messung eine erste Impedanzmessung unter Verwendung eines ersten Elektrodenpaares der mehreren Elektroden und eine zweite Impedanzmessung unter Verwendung eines zweiten Elektrodenpaares der mehreren Elektroden beinhaltet; und
wobei die zweite Messung eine dritte Impedanzmessung unter Verwendung des ersten Elektrodenpaares und eine vierte Impedanzmessung unter Verwendung des zweiten Elektrodenpaares beinhaltet.

4. Medizinische Behandlungsvorrichtung nach einem der Ansprüche 1 bis **3,**

wobei die erste Messung eine erste Temperaturmessung und eine erste Impedanzmessung beinhaltet; und

wobei die zweite Messung eine zweite Temperaturmessung und eine zweite Impedanzmessung beinhaltet.

5. Medizinische Behandlungsvorrichtung nach einem der Ansprüche 1 bis **4,** ferner umfassend eine Herzzyklus-Überwachungsvorrichtung,

wobei die Verarbeitungsschaltung ferner dazu ausgelegt ist, die therapeutische Wirkung basierend auf einer oder mehreren Herzzyklusphasen zu schätzen, die aus einer hieraus bestehenden Gruppe ausgewählt wird/werden:

einer ersten Herzzyklusphase, die der ersten Messung entspricht;
einer zweiten Herzzyklusphase, die der jeweiligen Zeitinstanz entspricht; und
einer dritten Herzzyklusphase, die der zweiten Messung entspricht.

6. Medizinische Behandlungsvorrichtung nach einem der Ansprüche 1 bis 5, ferner umfassend einen Wellenformgenerator, der in elektrischer Kommunikation mit den mehreren Elektroden steht, um auf diese eine Folge von Pulsen der PEF-Energie anzuwenden,

wobei die Verarbeitungsschaltung ferner dazu ausgelegt ist, die therapeutische Wirkung basierend auf einer Zeitverzögerung zwischen einem Paar von aufeinanderfolgenden Pulsen der PEF-Energie in der Folge von Pulsen zu schätzen.

7. Medizinische Behandlungsvorrichtung nach einem der Ansprüche 1 bis 6, wobei die Verarbeitungsschaltung ferner dazu ausgelegt ist, die therapeutische Wirkung basierend auf einer kumulativen Vollständigkeitsmessung zu schätzen, die als eine gewichtete Summe von mehreren unterschiedlichen Einzelmessungen der Vollständigkeit berechnet wird, insbesondere

wobei die mehreren unterschiedlichen Einzelmessungen der Vollständigkeit zwei oder mehr Einzelmessungen der Vollständigkeit beinhalten, die aus einer hieraus bestehenden Gruppe ausgewählt werden:

einem Paar von Einzelmessungen der Vollständigkeit, die unterschiedlichen jeweiligen Zeitinstanzen der PEF-Energieabgabe entsprechen;
einem Paar von Einzelmessungen der Vollständigkeit, die unterschiedlichen jeweiligen Arten von gemessenen Werten entsprechen;
einem Paar von Einzelmessungen der Vollständigkeit, die unterschiedlichen jeweiligen Untermengen der mehreren Elektroden entsprechen; und
einem Paar von Einzelmessungen der Vollständigkeit, die unterschiedlichen jeweiligen Sätzen von Verschiebungswerten entsprechen.

8. Medizinische Behandlungsvorrichtung nach einem der Ansprüche 1 bis 7, wobei die Verarbeitungsschaltung Schaltungen zum Generieren einer in Zonen eingeteilten PEF-Energieauswirkungskarte für einen einzelnen PEF-Energieanwendungspunkt beinhaltet, welcher der jeweiligen Zeitinstanz basierend auf der ersten Messung, der zweiten Messung, der ersten Verschiebung und der zweiten Verschiebung entspricht, insbesondere wobei die Schaltungen dazu ausgelegt sind, eine Behandlungsvollständigkeitskarte zu generieren, indem mehrere in Zonen eingeteilte PEF-Energieauswirkungskarten, die unterschiedlichen einzelnen PEF-Energieanwendungspunkten entsprechen, kombiniert werden.

9. Therapiebeurteilungsverfahren, umfassend:

Empfangen, mit einer Verarbeitungsschaltung, eines Stroms von Therapiebeurteilungsmessungen, die mit mehreren Elektroden eines Katheters erhalten werden, der dazu ausgelegt ist, Energie eines gepulsten elektrischen Feldes (PEF) an ein Zielgewebe abzugeben, wobei die Therapiebeurteilungsmessungen eine erste Messung beinhalten, die vor einer jeweiligen Zeitinstanz einer PEF-Energieabgabe vorgenommen wird, und eine zweite Messung, die nach der jeweiligen Zeitinstanz vorgenommen wird;
Bestimmen, mit der Verarbeitungsschaltung, einer ersten Verschiebung der mehreren Elektroden zwischen der ersten Messung und der jeweiligen Zeitinstanz und einer zweiten Verschiebung der mehreren Elektroden zwischen der jeweiligen Zeitinstanz und der zweiten Messung, wobei das Bestimmen auf Positionsmessungen basiert, die von einem Verfolgungssystem empfangen werden, das dazu ausgelegt ist, eine Position einer ausgewählten Elektrode der mehreren Elektroden oder eines ausgewählten Verfolgungselements zu verfolgen; und
Schätzen, mit der Verarbeitungsschaltung, einer therapeutischen Wirkung der PEF-Energieabgabe basierend auf der ersten Messung, der zweiten Messung, der ersten Verschiebung und der zweiten Verschiebung.

10. Therapiebeurteilungsverfahren nach Anspruch 9, wobei das Schätzen ferner auf einer oder mehreren Herzzyklusphasen basiert, die aus einer hieraus bestehenden Gruppe ausgewählt wird/werden:

   einer ersten Herzzyklusphase, die der ersten Messung entspricht;
   einer zweiten Herzzyklusphase, die der jeweiligen Zeitinstanz entspricht; und
   einer dritten Herzzyklusphase, die der zweiten Messung entspricht.

11. Therapiebeurteilungsverfahren nach Anspruch 9 oder 10, wobei das Schätzen ferner auf einer Zeitverzögerung zwischen einem Paar von aufeinanderfolgenden Pulsen der PEF-Energie in einer Folge von Pulsen basiert, die von einem Wellenformgenerator auf die mehreren Elektroden angewendet wird, und/oder
   wobei das Schätzen ferner auf einer kumulativen Vollständigkeitsmessung basiert, die als eine gewichtete Summe von mehreren unterschiedlichen Einzelmessungen der Vollständigkeit berechnet wird.

12. Therapiebeurteilungsverfahren nach einem der Ansprüche 9 bis 11, wobei das Schätzen ferner auf einer Zeitverzögerung zwischen einem Paar von aufeinanderfolgenden Pulsen der PEF-Energie in einer Folge von Pulsen basiert, die von einem Wellenformgenerator auf die mehreren Elektroden angewendet wird.

13. Therapiebeurteilungsverfahren nach einem der Ansprüche 9 bis 12, wobei das Schätzen ferner auf einer kumulativen Vollständigkeitsmessung basiert, die als eine gewichtete Summe von mehreren unterschiedlichen Einzelmessungen der Vollständigkeit berechnet wird, insbesondere
   wobei die mehreren unterschiedlichen Einzelmessungen der Vollständigkeit zwei oder mehr Einzelmessungen der Vollständigkeit beinhalten, die aus einer hieraus bestehenden Gruppe ausgewählt werden:

   einem Paar von Einzelmessungen der Vollständigkeit, die unterschiedlichen jeweiligen Zeitinstanzen der PEF-Energieabgabe entsprechen;
   einem Paar von Einzelmessungen der Vollständigkeit, die unterschiedlichen jeweiligen Arten von gemessenen Werten entsprechen;
   einem Paar von Einzelmessungen der Vollständigkeit, die unterschiedlichen jeweiligen Untermengen der mehreren Elektroden entsprechen; und
   einem Paar von Einzelmessungen der Vollständigkeit, die unterschiedlichen jeweiligen Sätzen von Verschiebungswerten entsprechen.

14. Therapiebeurteilungsverfahren nach einem der Ansprüche 9 bis 13, ferner umfassend das Generieren, mit der Verarbeitungsschaltung, einer in Zonen eingeteilten PEF-Energieauswirkungskarte für einen einzelnen PEF-Energieanwendungspunkt, welcher der jeweiligen Zeitinstanz basierend auf der ersten Messung, der zweiten Messung, der ersten Verschiebung und der zweiten Verschiebung entspricht, insbesondere
   ferner umfassend das Generieren, mit der Verarbeitungsschaltung, einer Behandlungsvollständigkeitskarte, indem mehrere in Zonen eingeteilte PEF-Energieauswirkungskarten, die unterschiedlichen einzelnen PEF-Energieanwendungspunkten entsprechen, kombiniert werden.

15. Nicht-transitorisches computerlesbares Medium, auf dem Anweisungen gespeichert sind, die, wenn sie von einem elektronischen Prozessor ausgeführt werden, den elektronischen Prozessor veranlassen, Operationen gemäß dem Verfahren nach einem der Ansprüche 9 bis 14 durchzuführen.

**Revendications**

1. Appareil de traitement médical, comprenant :

   un cathéter comportant une pluralité d'électrodes configurées pour administrer une énergie de champ électrique pulsé (PEF) à un tissu cible et configurées en outre pour prendre des mesures d'évaluation de thérapie dans le tissu cible, comprenant une première mesure et une deuxième mesure ;
   un système de suivi configuré pour suivre une position d'une électrode sélectionnée parmi la pluralité d'électrodes ou d'un élément de suivi sélectionné afin de déterminer un premier déplacement de la pluralité d'électrodes entre la première mesure et un instant respectif de fourniture d'énergie PEF et un deuxième déplacement de la pluralité d'électrodes par rapport au tissu cible entre l'instant respectif et la deuxième mesure ; et
   un circuit de traitement configuré pour estimer un effet thérapeutique de l'administration d'énergie PEF sur la base de la première mesure, de la deuxième mesure, du premier déplacement et du deuxième déplacement,

la première mesure étant prise avant l'instant respectif ; et
la deuxième mesure étant prise après l'instant respectif.

2. Appareil de traitement médical selon la revendication 1,

la première mesure comprenant une première mesure de température utilisant une première électrode de la pluralité d'électrodes et une deuxième mesure de température utilisant une deuxième électrode de la pluralité d'électrodes ; et
la deuxième mesure comprenant une troisième mesure de température utilisant la première électrode et une quatrième mesure de température utilisant la deuxième électrode.

3. Appareil de traitement médical selon la revendication 1 ou 2,

la première mesure comprenant une première mesure d'impédance utilisant une première paire d'électrodes de la pluralité d'électrodes et une deuxième mesure d'impédance utilisant une deuxième paire d'électrodes de la pluralité d'électrodes ; et
la deuxième mesure comprenant une troisième mesure d'impédance utilisant la première paire d'électrodes et une quatrième mesure d'impédance utilisant la deuxième paire d'électrodes.

4. Appareil de traitement médical selon l'une quelconque des revendications 1 à 3,

la première mesure comprenant une première mesure de température et une première mesure d'impédance ; et
la deuxième mesure comprenant une deuxième mesure de température et une deuxième mesure d'impédance.

5. Appareil de traitement médical selon l'une quelconque des revendications 1 à 4, comprenant en outre un moniteur de cycle cardiaque,
le circuit de traitement étant en outre configuré pour estimer l'effet thérapeutique sur la base d'une ou plusieurs phases de cycle cardiaque sélectionnées parmi le groupe constitué par :

une première phase de cycle cardiaque correspondant à la première mesure ;
une deuxième phase de cycle cardiaque correspondant à l'instant respectif ; et
une troisième phase de cycle cardiaque correspondant à la deuxième mesure.

6. Appareil de traitement médical selon l'une quelconque des revendications 1 à **5,** comprenant en outre un générateur de forme d'onde en communication électrique avec la pluralité d'électrodes pour y appliquer une séquence d'impulsions de l'énergie PEF,
le circuit de traitement étant en outre configuré pour estimer l'effet thérapeutique sur la base d'un retard entre une paire d'impulsions consécutives de l'énergie PEF dans la séquence d'impulsions.

7. Appareil de traitement médical selon l'une quelconque des revendications 1 à 6, le circuit de traitement étant en outre configuré pour estimer l'effet thérapeutique sur la base d'une mesure cumulative d'achèvement calculée comme une somme pondérée d'une pluralité de différentes mesures individuelles d'achèvement, en particulier
la pluralité de différentes mesures individuelles d'achèvement comprenant deux ou plus de deux mesures individuelles d'achèvement sélectionnées dans le groupe constitué par :

une paire de mesures individuelles d'achèvement correspondant à différents instants respectifs de l'administration d'énergie PEF ;
une paire de mesures individuelles d'achèvement correspondant à différents types respectifs de valeurs mesurées ;
une paire de mesures individuelles d'achèvement correspondant à différents sous-ensembles respectifs de la pluralité d'électrodes ; et
une paire de mesures individuelles d'achèvement correspondant à différents ensembles respectifs de valeurs de déplacement.

8. Appareil de traitement médical selon l'une quelconque des revendications 1 à 7, le circuit de traitement comprenant un circuit configuré pour générer une carte d'effet d'énergie PEF zonée pour un point d'application d'énergie PEF individuel correspondant à l'instant respectif sur la base de la première mesure, de la deuxième mesure, du premier déplacement et du deuxième déplacement, en particulier

les circuits étant configurés pour générer une carte d'achèvement du traitement en combinant une pluralité de cartes d'effet d'énergie PEF zonées correspondant à différents points d'application d'énergie PEF individuels.

9. Procédé d'évaluation d'une thérapie, comprenant :

la réception, avec un circuit de traitement, d'un flux de mesures d'évaluation de la thérapie obtenues avec une pluralité d'électrodes d'un cathéter configuré pour administrer une énergie de champ électrique pulsé (PEF) à un tissu cible, les mesures d'évaluation de la thérapie comprenant une première mesure prise avant un instant respectif d'administration de l'énergie PEF et une deuxième mesure prise après l'instant respectif ;

la détermination, à l'aide du circuit de traitement, d'un premier déplacement de la pluralité d'électrodes entre la première mesure et l'instant respectif et d'un deuxième déplacement de la pluralité d'électrodes entre l'instant respectif et la deuxième mesure, la détermination étant basée sur des mesures de position reçues d'un système de suivi configuré pour suivre une position d'une électrode sélectionnée parmi la pluralité d'électrodes ou d'un élément de suivi sélectionné ; et

l'estimation, à l'aide du circuit de traitement, d'un effet thérapeutique de l'administration d'énergie PEF sur la base de la première mesure, de la deuxième mesure, du premier déplacement et du deuxième déplacement.

10. Procédé d'évaluation thérapeutique selon la revendication 9, l'estimation étant en outre basée sur une ou plusieurs phases de cycle cardiaque sélectionnées parmi le groupe constitué par :

une première phase de cycle cardiaque correspondant à la première mesure ;
une deuxième phase de cycle cardiaque correspondant à l'instant respectif ; et
une troisième phase de cycle cardiaque correspondant à la deuxième mesure.

11. Procédé d'évaluation thérapeutique selon la revendication 9 ou 10, l'estimation étant en outre basée sur un retard entre une paire d'impulsions consécutives de l'énergie PEF dans une séquence d'impulsions appliquées à la pluralité d'électrodes par un générateur de formes d'onde, et/ou
l'estimation étant en outre basée sur une mesure cumulative d'achèvement calculée comme une somme pondérée d'une pluralité de mesures individuelles différentes d'achèvement.

12. Procédé d'évaluation thérapeutique selon l'une quelconque des revendications 9 à 11, l'estimation étant en outre basée sur un retard entre une paire d'impulsions consécutives de l'énergie PEF dans une séquence d'impulsions appliquées à la pluralité d'électrodes par un générateur de forme d'onde.

13. Procédé d'évaluation thérapeutique selon l'une quelconque des revendications 9 à 12, l'estimation étant en outre basée sur une mesure cumulative d'achèvement calculée comme une somme pondérée d'une pluralité de mesures individuelles différentes d'achèvement, en particulier
la pluralité de différentes mesures individuelles d'achèvement comprenant deux ou plus de deux mesures individuelles d'achèvement sélectionnées dans le groupe constitué par :

une paire de mesures individuelles d'achèvement correspondant à différents instants respectifs de l'administration d'énergie PEF ;
une paire de mesures individuelles d'achèvement correspondant à différents types respectifs de valeurs mesurées ;
une paire de mesures individuelles d'achèvement correspondant à différents sous-ensembles respectifs de la pluralité d'électrodes ; et
une paire de mesures individuelles d'achèvement correspondant à différents ensembles respectifs de valeurs de déplacement.

14. Procédé d'évaluation thérapeutique selon l'une quelconque des revendications 9 à 13, comprenant en outre la génération, avec le circuit de traitement, d'une carte d'effet d'énergie PEF zonée pour un point d'application d'énergie PEF individuel correspondant à l'instant respectif sur la base de la première mesure, de la deuxième mesure, du premier déplacement et du deuxième déplacement, en particulier
comprenant en outre la génération, avec le circuit de traitement, d'une carte d'achèvement du traitement en combinant une pluralité de cartes d'effet d'énergie PEF zonées correspondant à différents points d'application d'énergie PEF individuels.

15. Support non transitoire lisible par ordinateur stockant des instructions qui, lorsqu'elles sont exécutées par un processeur électronique, amènent le processeur électronique à effectuer des opérations comprenant le procédé selon l'une quelconque des revendications 9 à 14.

FIG. 1

EP 4 295 797 B1

Processing Circuitry *19*

Memory *23*

Processor *21*

Waveform Unit *25*

FIG. 2A

FIG. 2B

*28*
*28*
*33*
*28*
*28*
*31*
*28*

○ Application Point(s)

⊠ Irreversable Region

▨ Reversable Region

▨ Stimulation Region

FIG. 2C

*31*

▨ Completeness>90%
(I.e. Multiple Irreversable Field Exposures From Treatments)

▨ Completeness>80%
(I.e. Irreversable Field Exposures At Least Once)

▫ Completeness>40%
(I.e. Only Reversable Field Experienced

FIG. 2D

Application Point(s)

Irreversable Region

Reversable Region

Stimulation Region

28

Example: Ideal Field

FIG. 3A

37 35

28

Example: Some Uncertainty

FIG. 3B

28

Example: High Uncertainty

FIG. 3C

26

*100*

Measure A Position Of At Least One Of A Plurality Of Electrodes Of A Medical Device Prior To Or After Delivery Of PEF Energy To A Target Tissue With Respect To The Target Tissue (Step 102)

Deliver PEF Energy To The Target Tissue Sufficient To Electroporate At Least A Portion Of The Target Tissue (Step 104)

Determine At Least One Metric Of A Therapeutic Effect Based At Least In Part On The Measured Position (Step 106)

FIG. 4

FIG. 5

FIG. 6

FIG. 7

Catheter(s) — 710

Examples:
- Geometry
- Electrodes Engaged
- Bipolar/Monopolar
- Variable Engagement Routines

Target Tissue — 720

Examples:
- Atrial Cardiomyocytes
- Vetricular Cardiomyocytes
- Nerve
- Lung
- Cardiac Smooth Muscle
- Cardiac Scar Tissue

Therapy Parameters — 730

Examples:
- Voltage/Current
- Trains
- Pulses
- Rise/Fall Time
- Interphase
- Cycle
- Biphasic/Monophasic

Measurement — 740

Examples:
- Baseline Values
- Concurrent Therapy Measurement
- Post Therapy
- Post Therapy Initiation Time Series
- Position Information

Catheter Capability Dependance

Output — 760

Assessment Value

750

Examples:
- Temperature Change
- Impedance Change
- Time Dependent Rates (Temp,Z)
- Proximity

Target Values Lookup/Modeling — 754

Value(s)
Change(s)
Derivative(s)

$Value_1$ • • • $Value_n$

$C_{total}$

Matching Computation — 756

$C_i$

$w_i$

Examples:
- User Feedback
- EP Mapping System

$C_{total}$

(Optional) Filtering And Combination (E.g. For Multiple Targets) — 758

EP 4 295 797 B1

700

**EP 4 295 797 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020060757 A1

- US 20220273353